(19) Europäisches Patentamt
European Patent Office
Office européen des brevets

(11) **EP 4 640 161 A1**

(12) **EUROPEAN PATENT APPLICATION**

(43) Date of publication:
**29.10.2025 Bulletin 2025/44**

(21) Application number: **25167290.3**

(22) Date of filing: **31.03.2025**

(51) International Patent Classification (IPC):
**A61B 8/08** (2006.01)  **A61B 8/12** (2006.01)
**A61B 8/00** (2006.01)

(52) Cooperative Patent Classification (CPC):
**A61B 8/0841; A61B 8/12; A61B 8/4245;**
**A61B 8/5261**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **24.04.2024 US 202418645253**

(71) Applicant: **GE Precision Healthcare LLC**
**Waukesha, WI 53188 (US)**

(72) Inventors:
• **OLESEN, Jacob**
  **2730 Herlev (DK)**
• **JENSEN, Henrik**
  **2730 Herlev (DK)**
• **FRISVAD, Rasmus**
  **2730 Herlev (DK)**

(74) Representative: **Kilburn & Strode LLP**
**Lacon London**
**84 Theobalds Road**
**Holborn**
**London WC1X 8NL (GB)**

(54) **ULTRASOUND IMAGING BASED MEDICAL DEVICE TRACKING**

(57) A method includes transmitting an ultrasound signal, with an ultrasound probe (140), in a cavity of an object, receiving an echo signal, with the ultrasound probe, generating an image of an interior of the cavity, including a region of interest in the cavity, based on the echo signal, receiving a first signal from a first tracking sensor (156) of the ultrasound probe, receiving a second signal from a second tracking sensor (138) at a cannula (130) disposed in a wall of the object, receiving a third signal from a third tracking sensor (124) of a medical device (102), wherein part of a shaft (118) of the medical device is in the cannula and an instrument (134) disposed at an end region of the part of the shaft is in the cavity, and processing the first, second and third signals to estimate a first location of the instrument in the cavity relative to the region of interest.

FIGURE 1

## Description

FIELD

The following generally relates to ultrasound imaging, and finds particular application to ultrasound imaging based medical device tracking.

BACKGROUND

Ultrasound imaging provides real-time imaging of information about the interior of an object or a subject such as tissue, organs, etc. An example ultrasound imaging system generally includes an ultrasound imaging probe and a console. The ultrasound probe houses a transducer array, and the console includes or is in electrical communication with a display monitor and a user interface. The transducer array transmits a pressure wave and receives echoes produced in response to the pressure wave interacting with structure such as tissue, blood cells, etc. The echoes are converted to analog signals, which are amplified, digitized, and beamformed to produce scan lines of radio frequency (RF) data. The scan lines are processed (e.g., band-pass filtering, envelope detection, logarithmic compression, etc.), scan converted, and displayed as a 2-D (B-mode) ultrasound image.

A laparoscopic ultrasound imaging probe is configured to guide procedures performed in a cavity, such as the abdomen or pelvis, using small incisions (i.e., laparoscopic procedures), e.g., to guide a medical device to target tissue of interest for an ablation, biopsy, etc. An example of such a medical device includes a handle, a shaft and an instrument for the procedure at a distal end of the shaft. The probe is first employed to localize the target tissue of interest. This includes inserting the probe through an incision into the cavity and imaging the interior of the cavity to locate the target tissue of interest. The instrument of the medical device is then advanced through another incision to the target tissue of interest. A tracking system tracks the spatial location of both the ultrasound imaging probe and the medical device. For guidance, a graphical representation of an approximate location of the instrument is superimposed over the displayed ultrasound image.

The tracking system tracks the probe via a tracking sensor integrated in and/or disposed with the probe. In one approach, the tracking system tracks the instrument of the medical device via a tracking sensor disposed at a proximal end of the shaft of the medical device near the handle. With this approach, given a length of the medical device from handle to tip of the instrument, advancement of the instrument in the cavity is tracked by tracking movement of the tracking sensor outside of the cavity. For instance, where the tracking sensor indicates advancement of X centimeters (cm), the instrument is estimated to have linearly advanced X cm in the cavity. However, the shaft of the instrument is flexible and susceptible to deflection, and the mechanical load applied by an operator when advancing the medical device results in deflection of the shaft.

As a consequence, the estimated location of the instrument in the cavity (e.g., as displayed over the image) may not accurately reflect the actual location of the instrument in the cavity. An approach to address such deflection includes using a rigid cannula in the incision and advancing the shaft of the medical device through the rigid cannula, which limits deflection of the shaft, to the target tissue of interest. With this approach, the tracking sensor is disposed at the cannula, outside of the cavity, and the tracking sensor senses a direction of the advancement of the instrument. This approach may reduce overall deflection of the shaft as it is advanced in the cavity. However, with this approach, the tracking system is unable to track the location of the instrument. The tip of the instrument could be directly tracked with a tracking sensor, however, since instruments like needles are configured in different gages, this approach would depend on the choice of instrument.

In view of at least the foregoing, there is an unresolved need for an improved approach for ultrasound imaging based instrument tracking.

SUMMARY

Aspects of the application address the above matters, and others. This summary introduces concepts that are described in more detail in the detailed description. It should not be used to identify essential features of the claimed subject matter, nor to limit the scope of the claimed subject matter.

In one aspect, a method includes transmitting an ultrasound signal, with an ultrasound probe, in a cavity of an object. The method further includes receiving an echo signal, with the ultrasound probe. The method further includes generating an image of an interior of the cavity, including a region of interest in the cavity, based on the echo signal, receiving a first signal from a first tracking sensor of the ultrasound probe. The method further includes receiving a second signal from a second tracking sensor at a cannula disposed in a wall of the object. The method further includes receiving a third signal from a third tracking sensor of a medical device. Part of a shaft of the medical device is in the cannula and an instrument disposed at an end region of the part of the shaft is in the cavity. The method further includes processing the first, second and third signals to estimate a first location of the instrument in the cavity relative to the region of interest.

In one aspect, the method further comprises displaying the image, and superimposing indicia representing the instrument over the image based on the estimated first location where the instrument is in a plane of the image. In one aspect, the method further comprises superimposing indicia representing a trajectory from the instrument to the region of interest over the image. In one aspect, the method further comprises displaying the image, and superimposing indicia representing a trajectory from the instrument to the region of interest over the image based on the estimated first location where the instrument is outside of a plane of the image. In one aspect, the method further comprises determining a change in a location of the medical device based on the third sensor and estimating a new location of the instrument in the cavity based on the change in the position. In one aspect, the method further comprises determining a portion of the shaft between the second and third sensors includes a bend based on the second and third signals, and estimating the new location of the instrument in the cavity based on the change in location and the bend in the portion of the shaft. In one aspect, the method further comprises receiving a fourth signal from the second tracking sensor at the cannula disposed in the wall of the object, receiving a fifth signal from the third tracking sensor of the medical device, and processing the fourth and fifth signals to determine a second estimated location of the instrument in the cavity relative to the region of interest.

In another aspect, a computer readable medium encoded with computer executable instructions, which, when executed by a processor, cause the processor to: receive an echo signal with an ultrasound probe disposed in a cavity of an object, generate an image of an interior of the cavity, including a region of interest in the cavity, based on the echo signal, receive a first signal from a first tracking sensor of the ultrasound probe, receive a second signal from a second tracking sensor at a cannula disposed in a wall of the object, receive a third signal from a third tracking sensor of a medical device, wherein part of a shaft of the medical device is in the cannula and an instrument disposed at an end region of the part of the shaft is in the cavity, and process the first, second, and third signals to estimate a first location of the instrument in the cavity relative to the region of interest.

In one aspect, the instructions further cause the processor to display the image, and superimpose indicia representing the instrument over the image based on the estimated first location where the instrument is in a plane of the image. In one aspect, the instructions further cause the processor to superimpose indicia representing a trajectory from the instrument to the region of interest over the image. In one aspect, the instructions further cause the processor to display the image and superimpose indicia representing a trajectory from the instrument to the region of interest over the image based on the estimated first location where the instrument is outside of a plane of the image. In one aspect, the instructions further cause the processor to determine a change in a location of the medical device based on the third sensor, and estimate a new location of the instrument in the cavity based on the change in the position. In one aspect, the instructions further cause the processor to determine a portion of the shaft between the second and third sensors includes a bend based on the second and third signals, and estimate the new location of the instrument in the cavity based on the change in location and the bend in the portion of the shaft. In one aspect, the instructions further cause the processor to receive a fourth signal from the second tracking sensor at the cannula disposed in the wall of the object, receive a fifth signal from the third tracking sensor of the medical device, and process the fourth and fifth signals to determine a second estimated location of the instrument in the cavity relative to the region of interest.

In another aspect, a system includes an ultrasound imaging system configured to generate an image of an interior of a cavity of an object, wherein the ultrasound imaging system includes a probe. The system further includes a tracking system configured to receive a first signal from a first tracking sensor of the ultrasound probe, receive a second signal from a second tracking sensor at a cannula disposed in a wall of the object, and receive a third signal from a third tracking sensor of a medical device, wherein part of a shaft of the medical device is in the cannula and an instrument disposed at an end region of the part of the shaft is in the cavity. The system further includes a processor configured to process the first, second and third signals to estimate a first location of the instrument in the cavity relative to the region of interest.

In one aspect, the processor is further configured to display the image, and superimpose indicia representing the instrument over the image based on the estimated first location where the instrument is in a plane of the image. In one aspect, the processor is further configured to superimpose indicia representing a trajectory from the instrument to the region of interest over the image. In one aspect, the processor is further configured to display the image, and superimpose indicia representing a trajectory from the instrument to the region of interest over the image based on the estimated first location where the instrument is outside of a plane of the image. In one aspect, the processor is further configured to determine a change in a location of the medical device based on the third sensor, determine a bend in a portion of the shaft between the second and third sensors based on the second and third signals, and estimate a new location of the instrument in the cavity based on the change in location and the bend in the portion of the shaft. In one aspect, the processor is further configured to receive a fourth signal from the second tracking sensor at the cannula disposed in the wall of the object, receive a fifth signal from the third tracking sensor of the medical device, and process the fourth and fifth signals to determine a second estimated location of the instrument in the cavity relative to the region of interest.

Those skilled in the art will recognize still other aspects of the present application upon reading and understanding the attached description.

BRIEF DESCRIPTION OF THE DRAWINGS

The application is illustrated by way of example and not limited by the figures of the accompanying drawings in which like references indicate similar elements.

FIGURE 1 schematically illustrates a non-limiting example that includes an ultrasound imaging system, a medical device, a wireless tracking system, and an image guidance system that includes a tracking module, in accordance with an aspect of an embodiment(s) herein.

FIGURE 2 schematically illustrates a non-limiting example of a probehead of a probe of the ultrasound imaging system of FIGURE 1, in accordance with an aspect of an embodiment(s) herein.

FIGURE 3 schematically illustrates another example in which the ultrasound imaging system includes the tracking module and the image guidance system is omitted, in accordance with an aspect of an embodiment(s) herein.

FIGURE 4 schematically illustrates another example in which the tracking system includes a wired tracking system, in accordance with an aspect of an embodiment(s) herein.

FIGURE 5 schematically illustrates a prior approach showing example deflection of the shaft of the medical device while advancing the medical device in a cavity towards a target tissue of interest.

FIGURE 6 schematically illustrates an example in which a trocar is utilized to mitigate or reduce deflection of the shaft of the medical device while advancing the medical device in the cavity towards the target tissue of interest, in accordance with an aspect of an embodiment(s) herein.

FIGURE 7 schematically illustrates a non-limiting example where the medical device includes a tracking sensor, the trocar includes a tracking sensor and the shaft outside of the cavity has not deflected, in accordance with an aspect of an embodiment(s) herein.

FIGURE 8 schematically illustrates a non-limiting example where the medical device includes the tracking sensor, the trocar includes the tracking sensor and the shaft outside of the cavity is deflected, in accordance with an aspect of an embodiment(s) herein.

FIGURE 9 schematically illustrates a non-limiting example of a laparoscopic ultrasound probe that includes an articulating head, in accordance with an aspect of an embodiment(s) herein.

FIGURE 10 schematically illustrates a non-limiting example of the laparoscopic ultrasound probe of FIGURE 9 showing up and down movement of the articulating head, in accordance with an aspect of an embodiment(s) herein.

FIGURE 11 schematically illustrates a non-limiting example of the laparoscopic ultrasound probe of FIGURE 9 showing left and right movement of the articulating head, in accordance with an aspect of an embodiment(s) herein.

FIGURE 12 illustrates another non-limiting example of a flow chart for a computer-implemented method based at least on image characteristics, in accordance with an embodiment(s) herein.

FIGURE 13 illustrates an approach for determining a length of an instrument in a cavity where a portion of the instrument outside of the cavity is deflected and not straight, in accordance with an embodiment(s) herein.

DETAILED DESCRIPTION

Embodiments of the present disclosure will now be described, by way of example, with reference to the figures, in which a system, a method and/or a computer readable medium includes instructions for an ultrasound image based medical device tracking approach that utilizes a tracking sensor with the medical device, a tracking sensor with a trocar that provides a gateway for an instrument of the medical device to be advanced to a target tissue of interest in a cavity of an object, and a tracking sensor with the ultrasound imaging probe. As discussed above, by way of example, ultrasound imaging provides real-time imaging of information about the interior of an object or a subject such as tissue, organs, etc., and a laparoscopic ultrasound imaging probe is configured to guide procedures performed in a cavity, such as the abdomen or pelvis, using small incisions (i.e., laparoscopic procedures), e.g., to guide a medical device to target tissue of interest for an ablation, biopsy, etc.

With one existing tracking approach, a proximal end of a shaft of a medical device near a handle of the medical device is tracked and a current location of an instrument at a distal end of the shaft in the cavity is estimated based on the current location of the sensor. However, as discussed above, the shaft of the medical device is flexible and susceptible to deflection, and the mechanical load applied by an operator when advancing the medical device results in deflection of the shaft, resulting in an estimated location of the instrument in the cavity (e.g., as displayed over the image) that may not accurately reflect the actual location of the instrument in the cavity. Another tracking approach utilizes a rigid cannular to reduce such deflection and tracks the direction of the cannula to track the direction of the instrument. This approach may reduce overall deflection of the shaft as it is advanced in the cavity, however, the tracking system is unable to track the actual instrument.

Described herein is an approach which allows for mitigating deflection of the shaft as the shaft is advanced in the cavity, sensing the direction of the shaft as the shaft is advanced in the cavity, and sensing the location of the instrument as the shaft is advanced in the cavity. As such, in one instance, the approach described herein provides for accurate placement of the instrument at the target tissue of interest, relative to a configuration in which the system does not employ the approach described herein. This approach is agnostic to the choice of medical device as it is independent of characteristics of the medical device such as a gage of a needle instrument and can improve the accuracy of the instrument placement, which may affect the outcome of certain procedures such as an ablation of a tumor, etc.

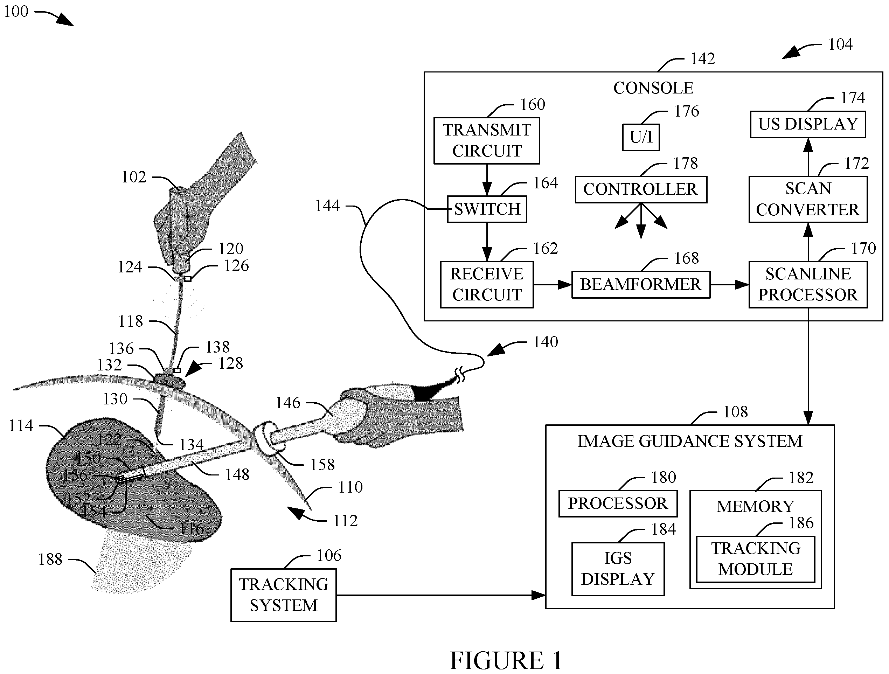

FIGURE 1 schematically illustrates a system 100 that includes a medical device 102, an ultrasound imaging system 104, a tracking system 106, and an image guidance system 108. For explanatory purposes, the instrument 102, ultrasound imaging system 104, the tracking system 106, and the image guidance system 108 are shown in connection with an object 110 having a cavity 112, a structure 114 in a cavity 112 of the object 110, and a target of interest 116 within the structure 114.

The medical device 102 includes an elongated shaft 118 having a long axis. A handle 120 is located at a first end of the elongated shaft 118. An instrument 122 is located at a second end of the elongated shaft 118, where the second end is opposite the first end, along the long axis of the elongated shaft 118. The instrument 122 is configured at least for ablation, biopsy, etc.

A sensor support 124 is removably disposed on the medical device 102. In the illustrated example, the sensor support 124 is disposed at a proximal end of the elongated shaft 118 by the handle 120. A tracking sensor 126 is removably disposed on the senser support 124, which carries the tracking sensor 126. Examples of the tracking sensor 126 include an electro-magnetic tracking device, an inertial tracking device, etc. In another instance, the sensor support 124 is omitted and the tracking sensor 126 is disposed directly on the elongated shaft 118 and/or the handle 120.

The medical device 102 is shown in connection with a trocar 128 in an incision in an outer wall of an object 110. The trocar 128 serves as a minimally invasive gateway for the medical device 102 to the target of interest 116 of the structure 114 in the cavity 112 of the object 110. The trocar 128 includes a rigid hollow elongated tube or cannula 130 having a long axis. The cannula 130 is disposed in the incision and the cavity 112. A port 132 is located at a first end of the cannula 130. The port 132 has a diameter larger than a diameter of the incision and is located outside of the object 110 at the incision. A tapered tip 134 is located at a second end of the cannula 130, where the second end is opposite the first end, along the long axis of the cannula 130.

A sensor support 136 is removably disposed at the port 132 of the trocar 128. A tracking sensor 138 is removably disposed on the sensor support 136, which carries the tracking sensor 138. Examples of the tracking sensor 138 include an electro-magnetic tracking device, an inertial tracking device, etc. In another instance, the sensor support 136 is omitted and the tracking sensor 138 is disposed directly at the port 132.

The ultrasound imaging system 104 includes a probe 140 and a console 142. The probe 140 and the console 142 interface with each other via a communication channel 144, which includes wired technology, e.g., complimentary interfaces and a cable therebetween (as illustrated), and/or wireless technology, e.g., Wi-Fi, etc. A handle 146 is located at a first end of the elongated shaft 148. A probehead 150 is located at a second end of the elongated shaft 148, where the second end is opposite the first end, along the long axis of the elongated shaft 148.

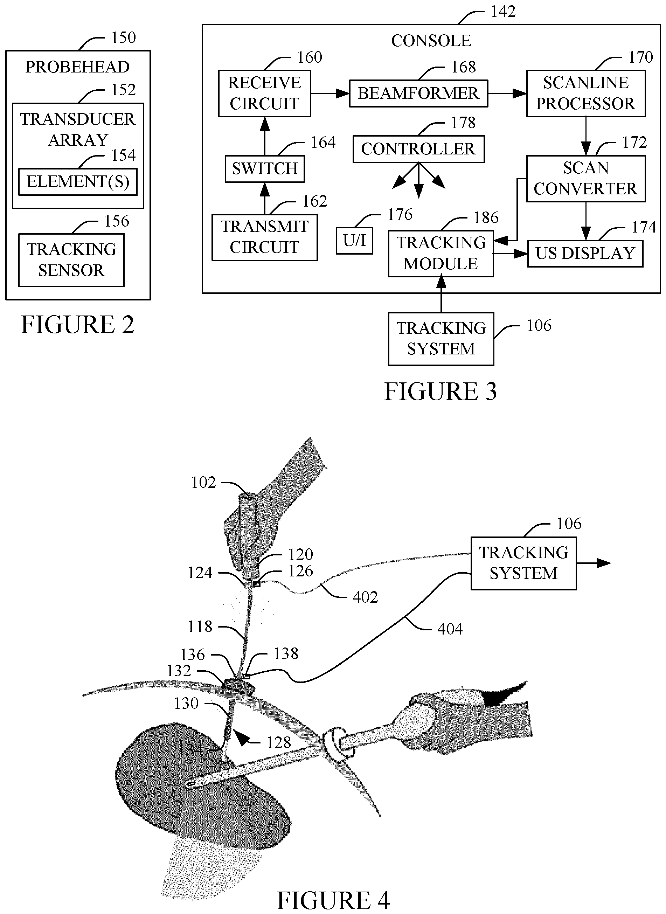

With continuing reference to FIGURE 1 and with reference to FIGURE 2, the probehead 150 houses a transducer array 152. The transducer array 152 includes one or more transducer elements 154. Examples of suitable arrays include 64, 128, 192, 256, and/or other arrays, including larger and smaller arrays, one dimensional (1-D) or two dimensional (2-D), etc. The transducer array 152 can be linear, curved, and/or otherwise shaped, fully populated, sparse and/or a combination thereof, etc. The one or more transducer elements 154 are configured to convert an excitation electrical signal to an ultrasound pressure field and convert a reflected ultrasound pressure field to an electrical signal.

By way of non-limiting example, the one or more transducer elements 154 can be selectively excited via an excitation electrical (pulsed) signal, which causes at least a sub-set of the transducer elements 154 to transmit an ultrasound pressure field into an examination or scan field of view. The ultrasound pressure field may include a focused ultrasound beam, a defocused (spherical) wave, and/or other ultrasound signal. The one or more transducer elements 154 receive echo signals and generate analog electrical signals indicative thereof. The echo signals are generated in response to the transmitted ultrasound pressure field interacting with structure, such as tissue and/or blood cells flowing in a portion of a vessel.

The probe 140 further includes a tracking sensor 156. The tracking sensor 156 is integrated in the probehead 150. Examples of the tracking sensor 156 include an electro-magnetic tracking device, an inertial tracking device, etc. In another instance, the tracking sensor 156 is disposed elsewhere in the probe 140.

The probe 140 is shown in connection with a trocar 158 in an incision in an outer wall of the object 110. The trocar 158 serves as a minimally invasive gateway for the probehead 150 and elongated shaft 148 of the probe 140 into the cavity 112 of the object 110. In another instance, the trocar 158 is omitted, and the probehead 150 and elongated shaft 148 of the probe 140 are inserted directly through the incision in the outer wall of the object 110.

With reference to FIGURE 1, the console 142 includes a transmit circuit 160 configured to generate the excitation electrical signal provided to transducer array 152 for transmitting the ultrasound pressure field. In one instance, this includes generating delays for individual elements 154 of the transducer array 152, e.g., for transmit focusing, beam steering, etc.

The console 142 further includes a receive circuit 162 configured to receive the analog electrical signals. In one instance, the receive circuit 162 is further configured to pre-process the analog electrical signals, e.g., amplify, digitize, focus, and/or otherwise process the analog electrical signals. For example, in one instance the receive circuit 162 includes an amplifier and a corresponding analog to digital converter (ADC) for each element, where each amplifier amplifies a corresponding analog electrical signal from a micro-volt level to a voltage range of the ADC.

The console 142 further includes a switch 164 configured to switch between the transmit circuit 160 and the receive circuit 162, e.g., by electrically connecting the transmit circuit 160 to the transducer array 152 for a transmit operation and electrically connecting the receive circuit 162 to the transducer array 152 for a receive operation. In an alternative instance, separate switches are employed for each of the transmit circuit 160 and the receive circuit 162.

The console 142 further includes a beamformer 168. For receive operations, the beamformer 168 is configured to beamform, e.g., via delay-and-sum (e.g., a matched-filter beamformer, etc.) and/or other beamforming, the signals from the receive circuit 162 and construct a scanplane of scanlines of radiofrequency (RF) data (RF signal) for the echoes for each receive operation. With delay-and-sum beamforming, the digital signal for each element is delayed to align the signals in time, amplified, and then summed. The output of the beamformer 168 includes the RF signal.

The console 142 further includes a scanline processor 170 configured to perform other processing on the data such as filtering (e.g., via a Finite Impulse Response (FIR) filter, an Infinite Impulse Response (IIR) filter, etc.), time gain compensation (TGC), I/Q demodulation, envelope detection, logarithmic compression, noise rejection, and/or other processing, and output frames of data. When configured for I/Q demodulation, the scanline processor 170 down mixes the RF signal and, optionally, apply low pass filtering and/or decimation. This may include employing a Hilbert Transform, a combination of a Complex-Demodulation Band Pass Filter and optional decimation, and/or other processing.

The scanline processor 170 detects and extracts the envelope (e.g., an amplitude) of the I/Q signal (when the scanline processor 170 I/Q demodulates the RF signal) or the RF signal (when the scanline processor 170 does not I/Q demodulate the RF signal). In one instance, this is achieved using a Hilbert transform and/or other approach. The scanline processor 170 compresses the extracted envelope, reducing the dynamic range thereof, e.g., to reduce the dynamic range to a predetermined display precision by a logarithmic (log)-based dynamic range compression and/or otherwise, and outputs a scanline. The scanline processor 170 outputs the processed scanlines as a frame / image (e.g., a B-mode image).

The console 142 further includes a scan converter 172. The scan converter 172 is configured to scan convert the image into a coordinate system of an ultrasound system (US) display 174. The scan converter 172 can be configured to employ analog and/or digital scan converting techniques. The ultrasound system display 174 is integrated with the console 142. In another instance, ultrasound system US display 174 is a separate and/or remote display monitor in electrical communication with the console 142.

The console 142 further includes a user interface (U/I) 176. The user interface 176 includes one or more input devices such as a button, a knob, a slider, a touch screen, a mouse, a keyboard, etc.) and/or other input device, and/or one or more output devices such as a visible, audible, etc. indicator. The user interface 176 allows a user to control an operation of the ultrasound imaging system 104. For example, in one instance, user interface 176 receives an input indicative of an imaging protocol including tracking of the instrument 122. The user interface 176 is shown integrated with the console 142. In another instance, the user interface 176 is a separate and/or remote keyboard, keypad, touch screen, etc. in electrical communication with the console 142.

The console 142 further includes a controller 178. The controller 178 includes a processor(s) such as a microprocessor ($\mu$P), a central processing unit (CPU), a graphics processing unit (GPU), etc., and memory, which stores the adaptive spatial compounding algorithm described herein. The controller 178 is configured to control one or more of the transmit circuit 160, the receive circuit 162, the switch 164, the beamformer 168, the scanline processor 170, the scan converter 172, the US display 174, and the user interface 176. One or more of the components of the console 142 can be implemented in software and/or hardware.

The tracking system 106 is described in the example in connection with passive electromagnetic tracking. With this example, the tracking system 106 includes a control unit and a transceiver. The sensors 126, 138 and 156 are each tuned at a certain resonance frequency. The transceiver is configured to emit an excitation signal including the resonance frequencies. The sensors 126, 138 and 156, in response to receiving signal, resonate at their tuned resonance frequency, emitting signals. The transceiver is further configured to receive the signals emitted by the sensors 126, 138 and 156. The control unit is configured to calculate a location and/or direction of each of the sensors 126, 138 and 156 in a common coordinate system based on the received signals, e.g., a strength of the signal. The tracking system 106 outputs the location and/or direction information.

The image guidance system 108 includes at least a processor 180 (e.g., a central processing unit, a microprocessor, etc.), a memory 182 (i.e., computer readable medium, which includes physical memory, etc., and excludes transitory medium such as a signal, carrier wave or other transitory medium), and an image guided system (IGS) display 184. The image guided system display 184 is shown integrated with the image guidance system 108. In another instance, the image guided system display 184 is a separate and/or remote display monitor in electrical communication with the image guidance system 108.

The memory 182 includes a tracking module 186 configured to process the location and/or direction information from the tracking system 106 and image from the scanline processor 170 of the ultrasound imaging system 104. In one instance, where the instrument 122 is in a plane 188 of the ultrasound beam, the tracking module 186 displays the image with graphical indicia at an estimated location of at least the instrument 122 superimposed or overlaid over the image, providing real-time display of images and the estimated location of at least the instrument 122. An example of suitable indicia includes a crosshair at an estimated location of a tip of the instrument 122. Another example additionally includes an end portion of the elongated shaft 118 along with the instrument 122.

In another instance, where the instrument 122 is not yet at the target of interest 116, the tracking module 186 superimposes graphical indicia representing an estimated trajectory from the estimated location of the instrument 122 to the target of interest 116. In another instance, where the instrument 122 is not yet in the plane 188 of the ultrasound beam, the tracking module 186 superimposes graphical indicia representing an estimated trajectory from the estimated location of the instrument 122 to the target of interest 116. The tracking module 186 updates the graphical indicia as the medical device 102, and, hence, the shaft 118 and the instrument are advanced in the cavity 112.

As described in greater detail below, the approached described herein, utilizing the tracking sensor 126 in connection with the instrument 102, the tracking sensor 138 in connection with the trocar 128, and the trocar 128 provides a more accurate estimated location of the instrument 122 of the medical device 102 in the cavity 112 of the object 110, relative to a configuration that does not utilize the tracking sensor 126 in connection with the instrument 102, the tracking sensor 138 in connection with the trocar 128. Again, this approach is agnostic to the choice of medical device as it is independent of characteristics of the medical device such as a gage of a needle instrument and may affect the outcome of certain procedures such as an ablation of a tumor, etc.

FIGURE 3 schematically illustrates another example of the system 100 in which the tacking module 186 is incorporated into the console 142. In one instance, the tracking module 186 is configured to process the location and/or direction information from the tracking system 106 and the image from the scan converter 172 (or just the location and/or direction information from the tracking system 106) and display the image with graphical indicia indicating an estimated location of at least the instrument 122 superimposed or overlaid over the image, as described herein, providing real-time display of images and the estimated location of at least the instrument 122.

FIGURE 4 schematically illustrates another example of the sensor 126 of the medical device 102 and the sensor 138 of the trocar 128. In FIGURE 1, the sensors 126 and the sensors 138 are passive sensors. In FIGURE 4, sensors 126 and the sensors 138 are active sensors. With this example, in one instance, the tracking system 106 includes a direct current (DC) or an alternating current (AC) field generator configured to produce an electromagnetic field that establishes a measurement volume, and the sensors 126 and 138 are in electrical communication with the tracking system 106 via respective electrical paths 402 and 404.

In this example, a generator of the tracking system 106 produces an electromagnetic field, and each of the sensors 126, 138 and 156, in response to the field, produces a signal, which are conveyed to the tracking system 106 via electrical paths. The tracking system 106 is configured to calculate a location and/or direction of each of the sensors 126, 138 and 156 in the field volume based on the received signals. The tracking system 106 provides the location and/or orientation information to the image guided guidance system 108 (FIGURE 1) and/or the console 142 (FIGURE 3).

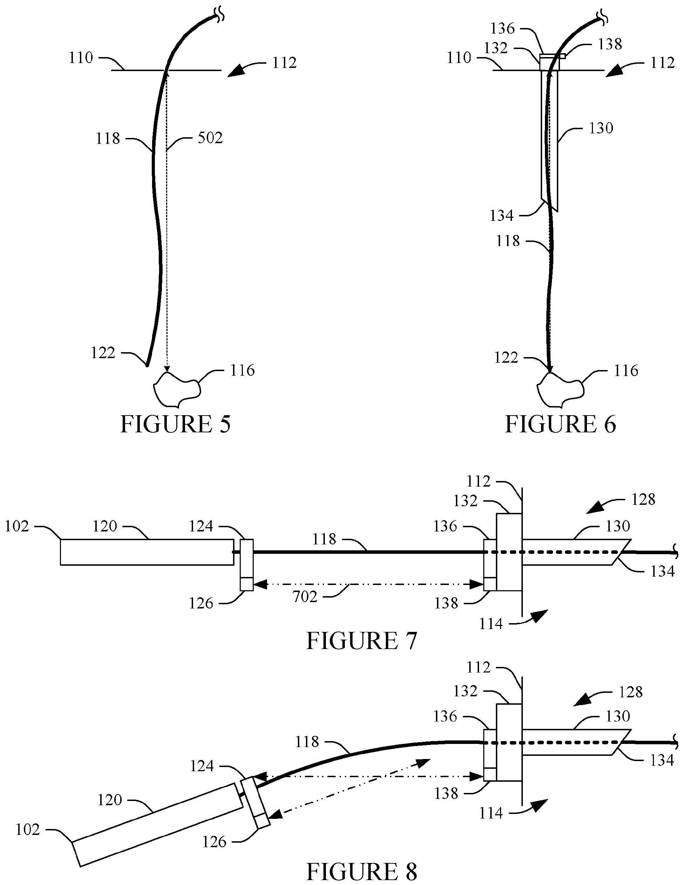

FIGURES 5 and 6 illustrate example deflection of the shaft 118 respectively without the trocar 128(FIGURE 5) and with the trocar 128 (FIGURE 6). In both FIGURES 5 and 6, a length of the shaft 118 is a same length (e.g., thirty to thirty-five centimeters (30-35 cm), etc.). FIGURE 5 shows a linear trajectory 502 in the cavity 112 presuming no deflection. FIGURE 5 further shows example deflection of the shaft 118 in the cavity 112. As a consequence, the instrument 122 is estimated to be at the target of interest 116, even though the time of the instrument 122 is displaced from the target of interest 116.

In FIGURE 6, the cannular 130 limits deflection of the shaft 118 at least over a length of the cannula 130. By way of non-limiting example, where the length of the cannula 130 is nine centimeters (9 cm), an amount of deflection of the shaft 118 for the first 9 cm is limited to a radius of the cannula 130. In another non-limiting example, where the length of the cannula 130 is fifteen centimeters (15 cm), an amount of deflection of the shaft 118 for the first 15 cm is limited to a radius of the cannula 130. In both instances, a distance between the tip 134 of the trocar 128 and the target of interest 116 is less than a distance between the incision and the target of interest 116.

As such, with the approached described herein, which utilizes the trocar 128, the shaft 118, in general, is less susceptible to greater deflection than when the trocar 128 is omitted. In addition, the tracking sensor 138 disposed on the trocar 128 provides information such as a direction at which the instrument 122 and the shaft 118 enter the cavity 112. As such, the approach described herein provides a more accurate estimation of the location of the instrument 122 relative to a configuration that omits the trocar 128.

FIGURES 7 and 8 extend the example of FIGURE 6, further showing the sensor support 124 and the sensor 126 near the handle 120 of the medical device 102, along with the trocar 128, the sensor support 136 and the sensor 138. The sensor 126 tracks the medical device 102. The information from the sensor 126 and the sensor 138 is also used to determine if the portion of the shaft 118 outside of the cavity 112 is straight or deflected. In FIGURE 7, the portion of the shaft 118 outside of the cavity 112 is straight, as indicated by a straight dashed and dotted line 702 parallel to the shaft 118 and between the sensor 126 and the sensor 138. As such, where the tracking sensor 126 indicates the medical device 102 advanced X cm, the tracking module 186 determines that the instrument 122 advanced X cm in the cavity 112 from the last estimated location.

FIGURE 8 shows an instance where the portion of the shaft 118 outside of the cavity 112 deflected. In FIGURE 8, the mechanical load applied by the operator to the medical device 102 resulted in deflection of the portion of the shaft 118 outside of the cavity 112 without advancing the portion of the shaft 118 inside of the cavity 112 and the instrument. The sensor 126 is closer to the object 110 relative to the sensor 126 in FIGURE 7, indicating the medical device 102 has advanced. However, in this instance, the information from the sensor 126 and the sensor 138 indicates that the portion of the shaft 118 outside of the cavity 112 deflected. For example, the sensors 126 and 138 are no longer aligned with each other. The information from the sensor 126 and the sensor 138 is utilized to determine a length of the portion of the shaft 118 outside of the cavity 112.

In the illustrated example, where the portion of the shaft 118 inside of the cavity 112 and the instrument, the portion of the shaft 118 outside of the cavity 112 is determined to be the same length as the portion of the shaft 118 outside of the cavity 112 in FIGURE 7, the tracking module 186 determines that the location of the instrument 122 is the same, even though the sensor 126 along indicates possible advancement of the portion of the shaft 118 inside of the cavity 112 and the instrument 122. In another example, the portion of the shaft 118 outside of the cavity 112 deflects and the portion of the shaft 118 inside of the cavity 112 advances. Likewise, the tracking sensor 126 determines the actual amount that the portion of the shaft 118 outside of the cavity 112 advanced (considering deflection) and estimates the location of the instrument 122 in the cavity 112 based thereon.

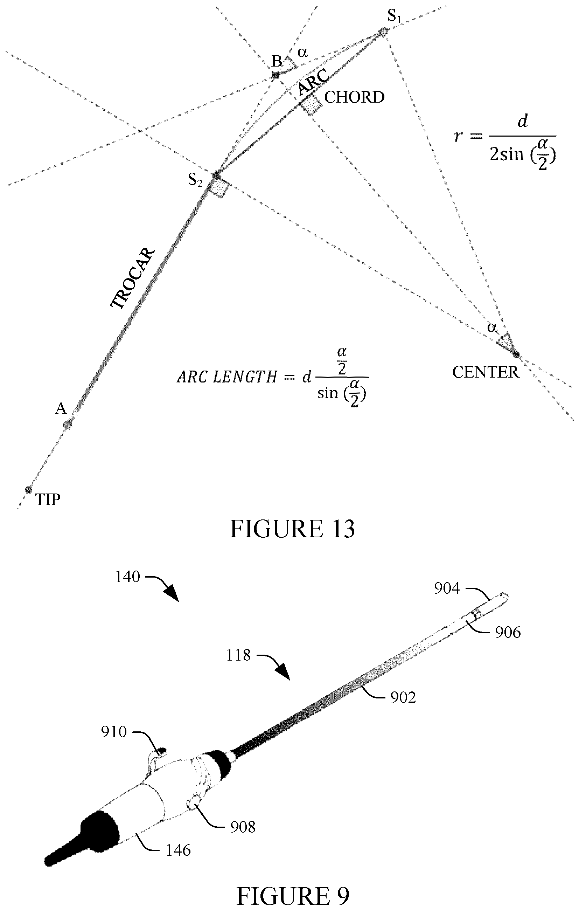

By way of non-limiting example, in one instance the instrument 122 is presumed to lie on a straight line between the sensor 126 and the sensor 136. The tracking module 186 calculates a distance between the sensors 126 and 136 by subtracting position data from the two sensors 126 and 136 from each other. A resulting distance is then subtracted from the total length of the instrument 122, which indicates a length of the instrument 122 in the body inside the cavity 112. For example, where a total length of the instrument 122 is 30 cm and there is a 5 cm distance between the two sensors 126 and 136, a length of the instrument 122 in the cavity 112 is 25 cm. In another example, any arc of the instrument 122 is taken into account. In this example, a length is determined as shown in connection with FIGURE 13 and below in EQUATION 1:

$$\text{EQUATION 1}$$
$$l = \frac{d}{sinc(\alpha/2)},$$

where l represents a curved length between the two sensors 126 and 136, d represents a shortest length between the two sensors 126 and 136, and $\alpha$ represents an angle between the two sensors 126 and 136.

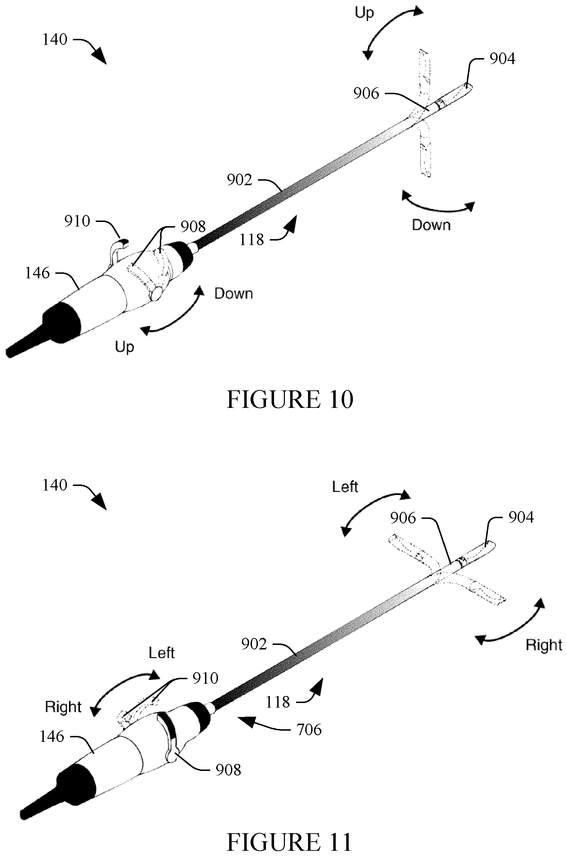

FIGURES 9, 10 and 11 illustrate an example of the probe 140 configured as an articulating laparoscopic probe. FIGURE 9 illustrates a side view of the probe 140. FIGURE 10 illustrates a perspective view of the probe 140 showing up and down articulation. FIGURE 11 illustrates a perspective view of the probe 140 showing right and left articulation.

The laparoscopic probe 140 includes the handle 146 and the elongated shaft 118. The elongated shaft 118 includes a body 902 and a head 904, both aligned along the long axis. The body 902 includes a first end and a second opposing end. The first end of the body 902 is affixed to the handle 146. An articulating member 906 couples the second opposing end of the body 902 and the head 904. In the illustrated embodiment, the articulating member 906 articulates in at least four directions (up, down, right and left). In a variation, the articulating member 906 articulates in at least two directions. In a variation, the articulating member 906 is omitted and the shaft 118 does not articulate. The handle 146 includes a first actuator 908 and a second actuator 910. The first actuator 908 actuates the articulating member 906 to control up/-down movement of the head 904. The second actuator 910 actuates the articulating member 906 to control left/-

right movement of the head 904.

An example of such a probe is the I12C4f 4-way laparoscopic probe, a product of B-K Medical ApS, a company of Herlev, Denmark, which is a wholly owned subsidiary of General Electric Healthcare, a company of WI, USA. Other laparoscopic probes are also contemplated herein. Examples of approaches for articulating a head of a transducer probe is described in patent US 9,897,694 B2, filed July 12, 2011, and entitled "Ultrasound Imaging Probe," and patent application US 10,383,598 B2, filed January 14, 2013, and entitled "Ultrasound Imaging Probe," both of which are incorporated herein by reference in their entireties.

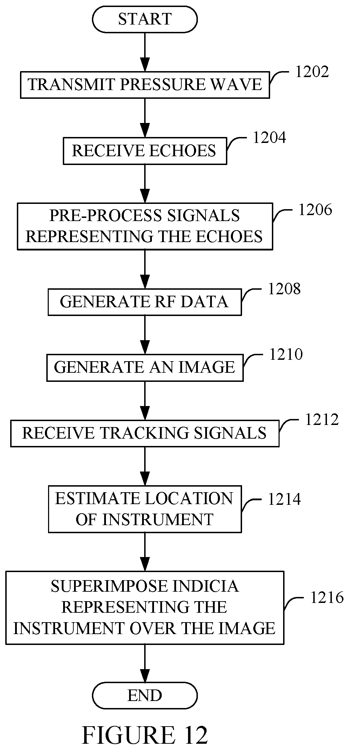

FIGURE 12 illustrates a non-limiting example of a flow chart for a computer-implemented method based at least on speckle statistics. It is to be appreciated that the ordering of the acts in the method is not limiting. As such, other orderings are contemplated herein. In addition, one or more acts may be omitted, and/or one or more additional acts may be included.

At 1202, the transducer array 152 is excited to emit a pressure wave, as described herein and/or otherwise. For example, the transmit circuit 160 generates and transmits an excitation electrical signal that excites at least a sub-set (i.e., not all) of the transducer elements 154, including generating delays for transducer elements 154 of the transducer array 152, e.g., for transmit focusing, beam steering, etc., which causes at least a sub-set of the transducer elements 154 to transmit an ultrasound pressure field into an examination or scan field of view.

At 1204, the at least a sub-set of the transducer elements 154 receive echo signal generated in response to the transmitted ultrasound pressure field interacting with structure, such as tissue and/or blood cells, and generates an analog signal indicative thereof, as described herein and/or otherwise. At 1206, the receive circuit 162 pre-processes the analog signal indicative, as described herein and/or otherwise. For example, in one instance the receive circuit 162 is configured to amplify, digitize, focus, etc. the analog electrical signals. For example, in one instance the receive circuit 162 includes an amplifier and a corresponding analog to digital converter (ADC) for each element, where each amplifier amplifies a corresponding analog electrical signal from a micro-volt level to a voltage range of the ADC.

At 1208, the beamformer 168 processes the digitized signals, as described herein and/or otherwise. For example, in one instance the beamformer 168 is configured to apply delay-and-sum and/or other beamforming, to the digitized signals and construct a scanplane of scanlines of radiofrequency (RF) data (RF signal) for the echoes for each receive operation, where for delay-and-sum beamforming, the digital signal for each element is delayed to align the signals in time, amplified, and then summed. At 1210, the scanline processor 170 and the scan converter 172 process the RF data and generates an image, as described herein and/or otherwise. This may include performing processing on the data such as filtering, TGC, I/Q demodulation, envelope detection, logarithmic compression, noise rejection, etc., and converting the resulting image into a coordinate system of an ultrasound system display 174.

At 1212, the tracking system 106 receives a signal from the tracking sensor 156 of the ultrasound probe, receives a signal from the tracking sensor 138 at trocar 128 and receives a signal from the tracking sensor 126 of the medical device 102, and conveys the signals to the tracking module 186 (which in the image guidance system 108 and/or the console 142 of the ultrasound imaging system 104), as described herein and/or otherwise. At 1214, the tracking module 186 processes the signals from the tracking sensors 126, 138 and 156 and estimates a first location of the instrument 122 in the cavity 112 relative to the target of interest 116 based on the signals from the tracking sensors 126, 138 and 156, as described herein and/or otherwise.

At 1216, the tracking module 186 processes the location and/or direction information and image from the scanline processor 170 and displays the image with graphical indicia at an estimated location of at least the instrument 122 superimposed or overlaid over the image, providing real-time display of images and the estimated location of at least the instrument 122, as described herein and/or otherwise. For example, this may include superimposing indicia representing a tip of the instrument 122 in relation to the target of interest 116 where the instrument is in the image plane 188, additionally or alternatively superimposing indicia representing a portion of the shaft at which the instrument 122 is disposed, superimposing indicia representing a trajectory from the instrument 122 to the target of interest 116, etc.

The above can be implemented by way of computer readable instructions, encoded, or embedded on the computer readable storage medium, which, when executed by a computer processor, cause the processor to carry out the described acts or functions. Additionally, or alternatively, at least one of the computer readable instructions is carried out by a signal, carrier wave or other transitory medium, which is not computer readable storage medium.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include such additional elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

The various embodiments and/or components, for example, the modules, or components and controllers therein, also may be implemented as part of one or more computers or processors. The computer or processor may include a computing device, an input device, a display unit and an interface, for example, for accessing the Internet. The computer or processor may include a microprocessor. The microprocessor may be connected to a communication bus. The computer or processor may also include a memory. The memory may include Random Access Memory (RAM) and Read Only Memory (ROM). The computer or processor further may include a storage device, which may be a hard disk drive or a removable storage drive such as a floppy disk drive, optical disk drive, and the like. The storage device may also be other similar means for loading computer programs or other instructions into the computer or processor.

As used herein, the term "computer" or "module" may include any processor-based or microprocessor-based system including systems using microcontrollers, reduced instruction set computers (RISC), application specific integrated circuits (ASICs), logic circuits, and any other circuit or processor capable of executing the functions described herein. The above examples are exemplary only and are thus not intended to limit in any way the definition and/or meaning of the term "computer." The computer or processor executes a set of instructions that are stored in one or more storage elements, in order to process input data. The storage elements may also store data or other information as desired or needed. The storage element may be in the form of an information source or a physical memory element within a processing machine.

The set of instructions may include various commands that instruct the computer or processor as a processing machine to perform specific operations such as the methods and processes of the various embodiments of the invention. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description.

This written description uses examples to disclose the various embodiments of the invention, including the best mode, and also to enable any person skilled in the art to practice the various embodiments of the invention, including making and using any devices or systems and performing any incorporated methods. The patentable scope of the various embodiments of the invention is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims.

Embodiments of the present disclosure shown in the drawings and described above are example embodiments only and are not intended to limit the scope of the appended claims, including any equivalents as included within the scope of the claims. Various modifications are possible and will be readily apparent to the skilled person in the art. It is intended that any combination of non-mutually exclusive features described herein are within the scope of the present disclosure. That is, features of the described embodiments can be combined with any appropriate aspect described above and optional features of any one aspect can be combined with any other appropriate aspect. Similarly, features set forth in dependent claims can be combined with non-mutually exclusive features of other dependent claims, particularly where the dependent claims depend on the same independent claim. Single claim dependencies may have been used in practice as some jurisdictions require them, but this should not be taken to mean that the features in the dependent claims are mutually exclusive.

## Claims

1.   A method, comprising:

transmitting an ultrasound signal, with an ultrasound probe (140), in a cavity of an object;

receiving an echo signal, with the ultrasound probe;

generating an image of an interior of the cavity, including a region of interest in the cavity, based on the echo signal;

receiving a first signal from a first tracking sensor (156) of the ultrasound probe;

receiving a second signal from a second tracking sensor (138) at a cannula (130) disposed in a wall of the object;

receiving a third signal from a third tracking sensor (124) of a medical device (102), wherein part of a shaft (118) of the medical device is in the cannula and an instrument (134) disposed at an end region of the part of the shaft is in the cavity; and

processing the first, second and third signals to estimate a first location of the instrument in the cavity relative to the region of interest.

2. The method of claim 1, further comprising:

   displaying the image; and
   superimposing indicia representing the instrument over the image based on the estimated first location where the instrument is in a plane of the image.

3. The method of claim 2, further comprising:
   superimposing indicia representing a trajectory from the instrument to the region of interest over the image.

4. The method of claim 1, further comprising:

   displaying the image; and
   superimposing indicia representing a trajectory from the instrument to the region of interest over the image based on the estimated first location where the instrument is outside of a plane of the image.

5. The method of claim 1, further comprising:

   determining a change in a location of the medical device based on the third sensor; and
   estimating a new location of the instrument in the cavity based on the change in the position.

6. The method of claim 5, further comprising:

   determining a portion of the shaft between the second and third sensors includes a bend based on the second and third signals; and
   estimating the new location of the instrument in the cavity based on the change in location and the bend in the portion of the shaft.

7. The method of claim 1, further comprising:

   receiving a fourth signal from the second tracking sensor at the cannula disposed in the wall of the object;
   receiving a fifth signal from the third tracking sensor of the medical device, and
   processing the fourth and fifth signals to determine a second estimated location of the instrument in the cavity relative to the region of interest.

8. A computer readable medium encoded with computer executable instructions, which, when executed by a processor, cause the processor to:

   receive an echo signal with an ultrasound probe disposed in a cavity of an object;
   generate an image of an interior of the cavity, including a region of interest in the cavity, based on the echo signal;
   receive a first signal from a first tracking sensor of the ultrasound probe;
   receive a second signal from a second tracking sensor at a cannula disposed in a wall of the object;
   receive a third signal from a third tracking sensor of a medical device, wherein part of a shaft of the medical device is in the cannula and an instrument disposed at an end region of the part of the shaft is in the cavity; and
   process the first, second, and third signals to estimate a first location of the instrument in the cavity relative to the region of interest.

9. The computer readable medium of claim 8, wherein the instructions further cause the processor to:

   display the image; and
   superimpose indicia representing the instrument over the image based on the estimated first location where the instrument is in a plane of the image.

10. The computer readable medium of claim 9, wherein the instructions further cause the processor to:
    superimpose indicia representing a trajectory from the instrument to the region of interest over the image.

11. The computer readable medium of claim 8, wherein the instructions further cause the processor to:

    display the image; and
    superimpose indicia representing a trajectory from the instrument to the region of interest over the image based on the estimated first location where the instrument is outside of a plane of the image.

12. The computer readable medium of claim 8, wherein the instructions further cause the processor to:

determine a change in a location of the medical device based on the third sensor; and estimate a new location of the instrument in the cavity based on the change in the position.

13. The computer readable medium of claim 12, wherein the instructions further cause the processor to:

determine a portion of the shaft between the second and third sensors includes a bend based on the second and third signals; and estimate the new location of the instrument in the cavity based on the change in location and the bend in the portion of the shaft.

14. The computer readable medium of claim 8, wherein the instructions further cause the processor to:

receive a fourth signal from the second tracking sensor at the cannula disposed in the wall of the object; receive a fifth signal from the third tracking sensor of the medical device, and process the fourth and fifth signals to determine a second estimated location of the instrument in the cavity relative to the region of interest.

15. A system, comprising:

an ultrasound imaging system (104) configured to generate an image of an interior of a cavity of an object, wherein the ultrasound imaging system includes a probe; a tracking system (106) configured to:

receive a first signal from a first tracking sensor of the ultrasound probe; receive a second signal from a second tracking sensor at a cannula disposed in a wall of the object; and receive a third signal from a third tracking sensor of a medical device, wherein part of a shaft of the medical device is in the cannula and an instrument disposed at an end region of the part of the shaft is in the cavity; and

a processor (180) configured to process the first, second and third signals to estimate a first location of the instrument in the cavity relative to the region of interest.

FIGURE 1

EP 4 640 161 A1

FIGURE 2

FIGURE 3

FIGURE 4

FIGURE 5

FIGURE 6

FIGURE 7

FIGURE 8

$$r = \frac{d}{2\sin\left(\frac{\alpha}{2}\right)}$$

$$ARC\ LENGTH = d\,\frac{\frac{\alpha}{2}}{\sin\left(\frac{\alpha}{2}\right)}$$

FIGURE 13

FIGURE 9

FIGURE 10

FIGURE 11

START

TRANSMIT PRESSURE WAVE — 1202

RECEIVE ECHOES — 1204

PRE-PROCESS SIGNALS
REPRESENTING THE ECHOES — 1206

GENERATE RF DATA — 1208

GENERATE AN IMAGE — 1210

RECEIVE TRACKING SIGNALS — 1212

ESTIMATE LOCATION
OF INSTRUMENT — 1214

SUPERIMPOSE INDICIA
REPRESENTING THE
INSTRUMENT OVER THE IMAGE — 1216

END

FIGURE 12

EP 4 640 161 A1

# EUROPEAN SEARCH REPORT

**Application Number**

EP 25 16 7290

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/159752 A1 (BHARAT SHYAM [US] ET AL) 30 May 2019 (2019-05-30)<br>* abstract *<br>* figures 1-7 *<br>* paragraph [0005] - paragraph [0044] *<br>- - - - - | 1-15 | INV.<br>A61B8/08<br>A61B8/12<br>A61B8/00 |
| A | CHAN ET AL: "A needle tracking device for ultrasound guided percutaneous procedures",<br>ULTRASOUND IN MEDICINE AND BIOLOGY, NEW YORK, NY, US,<br>vol. 31, no. 11,<br>2 November 2005 (2005-11-02), pages 1469-1483, XP005206656,<br>ISSN: 0301-5629, DOI:<br>10.1016/J.ULTRASMEDBIO.2005.07.014<br>* abstract *<br>* figures 1-10 *<br>* Section "Methods" *<br>- - - - - | 1-15 | |
| A | PENG CHANG ET AL: "Recent Advances in Tracking Devices for Biomedical Ultrasound Imaging Applications",<br>MICROMACHINES (BASEL), [Online]<br>vol. 13, no. 11,<br>29 October 2022 (2022-10-29), page 1855, XP093226750,<br>Switzerland<br>ISSN: 2072-666X, DOI: 10.3390/mi13111855<br>Retrieved from the Internet:<br>URL:https://www.mdpi.com/2072-666X/13/11/1855><br>[retrieved on 2025-08-19]<br>* abstract *<br>* figures 1-18 *<br>* Sections 1 - 5 *<br>- - - - - | 1-15 | **TECHNICAL FIELDS SEARCHED (IPC)**<br><br>A61B |

The present search report has been drawn up for all claims

| Place of search | Date of completion of the search | Examiner |
|---|---|---|
| Munich | 19 August 2025 | Moehrs, Sascha |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

19

## ANNEX TO THE EUROPEAN SEARCH REPORT
## ON EUROPEAN PATENT APPLICATION NO.

EP 25 16 7290

This annex lists the patent family members relating to the patent documents cited in  the above-mentioned European search report.
The members are as contained in the European Patent Office EDP file on
The European Patent Office is in no way liable for these particulars which are merely given for the purpose of information.

19-08-2025

| Patent document cited in search report | Publication date | Patent family member(s) | | Publication date |
|---|---|---|---|---|
| US 2019159752 A1 | 30-05-2019 | CN | 109152565 A | 04-01-2019 |
| | | EP | 3454757 A1 | 20-03-2019 |
| | | JP | 6664517 B2 | 13-03-2020 |
| | | JP | 2019514598 A | 06-06-2019 |
| | | US | 2019159752 A1 | 30-05-2019 |
| | | WO | 2017194314 A1 | 16-11-2017 |

EPO FORM P0459

For more details about this annex : see Official Journal of the European Patent Office, No. 12/82

**REFERENCES CITED IN THE DESCRIPTION**

**Patent documents cited in the description**

- US 9897694 B2

- US 10383598 B2